# EUROPÄISCHE PATENTSCHRIFT

(11) **EP 3 859 235 B1**
(45) Veröffentlichungstag und Bekanntmachung des Hinweises auf die Patenterteilung: **24.09.2025**
(21) Anmeldenummer: 20154912.8
(22) Anmeldetag: 31.01.2020
(51) Int. Cl.: F25B 1/10, F25B 9/00

(54) **PRÜFKAMMER UND VERFAHREN ZUR STEUERUNG**
TEST CHAMBER AND METHOD FOR CONTROLLING
CHAMBRE D'ESSAI ET PROCÉDÉ DE COMMANDE

(43) Veröffentlichungstag der Anmeldung: 04.08.2021
(73) Patentinhaber: Weiss Technik GmbH, 35447 Reiskirchen (DE)
(72) Erfinder: REUSCHEL, Dennis, 35396 Gießen (DE); STROH, Björn, 35329 Gemünden (DE)
(74) Vertreter: advotec.

(56) Entgegenhaltungen:
- EP-A1- 3 584 512
- JP-A- S5 442 058
- US-A- 2 434 221
- US-A- 5 062 274
- US-A1- 2018 328 628

## Beschreibung

Die Erfindung betrifft eine Prüfkammer, insbesondere Klimakammer zur Konditionierung von Luft, sowie ein Verfahren zur Konditionierung von Luft in einem gegenüber einer Umgebung verschließbaren und temperaturgedämmten Prüfraum einer Prüfkammer zur Aufnahme von Prüfgut, wobei mittels einer Kühleinrichtung einer Temperiervorrichtung der Prüfkammer, mit einem Kühlkreislauf mit einem Kältemittel, einem Wärmeübertrager im Prüfraum, einem Niederdruckverdichter und einem in einer Strömungsrichtung dem Niederdruckverdichter nachfolgenden Hochdruckverdichter, einem Gaskühler, einer Speichereinrichtung für Kältemittel und einem Expansionsventil eine Temperatur innerhalb des Prüfraums ausgebildet wird, wobei die Temperatur in dem Prüfraum mittels einer Steuervorrichtung der Prüfkammer gesteuert und/oder geregelt wird, wobei das Kältemittel über einen Bypass des Kühlkreislaufs an dem Niederdruckverdichter vorbei zu dem Hochdruckverdichter geleitet wird, wenn der Niederdruckverdichter abgeschaltet ist, wobei mittels eines in einer Strömungsrichtung nachfolgend dem Gaskühler angeordneten Hochdruckventils des Kühlkreislaufs gasförmiges und/oder flüssiges Kältemittel in die Speichereinrichtung dosiert wird, wobei die Speichereinrichtung über einen des Kühlkreislaufs an einer Mitteldruckseite des Kühlkreislaufs in der Strömungsrichtung vor dem Hochdruckverdichter und nachfolgend dem Niederdruckverdichter angeschlossen ist.

Derartige Prüfkammern werden regelmäßig zur Überprüfung von physikalischen und/oder chemischen Eigenschaften von Gegenständen, insbesondere Vorrichtungen eingesetzt. So sind Temperaturprüfschränke oder Klimaprüfschränke bekannt, innerhalb derer Temperaturen in einem Bereich von -70°°C bis +180°°C eingestellt werden können. Bei Klimaprüfschränken können ergänzend gewünschte Klimabedingungen eingestellt werden, denen dann die Vorrichtung bzw. das Prüfgut über einen definierten Zeitraum ausgesetzt wird. Eine Temperierung eines das zu prüfende Prüfgut aufnehmendem Prüfraums erfolgt regelmäßig in einem Umluftkanal innerhalb des Prüfraums. Der Umluftkanal bildet einen Luftbehandlungsraum im Prüfraum aus, in dem Wärmetauscher zur Erwärmung oder Kühlung der den Umluftkanal bzw. den Prüfraum durchströmenden Luft angeordnet sind. Dabei saugt ein Lüfter bzw. ein Ventilator die im Prüfraum befindliche Luft an und leitet sie im Umluftkanal zu den jeweiligen Wärmetauschern. Das Prüfgut kann so temperiert oder auch einem definierten Temperaturwechsel ausgesetzt werden. Während eines Prüfinterwalls kann dann beispielsweise eine Temperatur zwischen einem Temperaturmaximum und einem Temperaturminimum der Prüfkammer wechseln. Eine derartige Prüfkammer ist beispielweise aus der EP 0 344 397 A2 bekannt.

Das in einem Kühlkreislauf eingesetzte Kältemittel sollte ein relativ geringes CO₂-Äquivalent aufweisen, das heißt ein relatives Treibhauspotenzial oder auch Global Warming Potential (GWP) sollte möglichst gering sein, um eine indirekte Schädigung der Umwelt durch das Kältemittel bei Freisetzung zu vermeiden. Es ist daher auch bekannt Kohlenstoffdioxid (CO₂) bzw. Kohlendioxid als Reinstoffkältemittel zu verwenden. Kohlenstoffdioxid ist kostengünstig erhältlich, nicht brennbar und durch einen GWP von 1 im Wesentlichen umweltneutral. Kohlenstoffdioxid weist eine Gefriertemperatur bzw. Tripelpunkt von -56,6°C auf, was eine Erzielung von niedrigeren Temperaturen mit Kohlenstoffdioxid alleine nicht ermöglicht.

Weiter sind Kühleinrichtungen bekannt, die als sogenannte Boosteranlage ausgeführt sind. In einem Kühlkreislauf der Kühleinrichtungen ist stets ein Hochdruckverdichter einem Niederdruckverdichter in Reihe nachgeschaltet, so dass eine stufenweise Verdichtung des Kältemittels mit dem Niederdruckverdichter und nachfolgend mit dem Hochdruckverdichter erfolgt. Aufgrund der hohen Anforderungen an eine Temperaturregelung innerhalb des Temperaturbereichs des Prüfraums kommt es während eines Betriebs der Prüfkammer regelmäßig zu Schwankungen in einer Lastanforderung. Eine von den Verdichtern und dem Expansionsventil erzeugte Kälteleistung muss daher stufenlos regelbar sein. Gleichwohl ist es wünschenswert, dass die Verdichter, wenn es sich beispielsweise um Kompressoren handelt, nicht häufig eingeschaltet und ausgeschaltet werden, um eine Lebensdauer der Verdichter zu verlängern.

Um geringe Temperaturdifferenzen zwischen einer Ist-Temperatur und einer Soll-Temperatur auszugleichen, ist es bekannt einen von den Verdichtern bewirkten Massenstrom im Kühlkreislauf an dem Wärmeüberträger über einen Bypass vorbeizuführen, um ungünstige Lastfälle an den Verdichtern und damit ein häufiges ein- und ausschalten zu vermeiden. Nachteilig ist hier jedoch, dass bei einer geringen Temperaturdifferenz am Wärmeübertrager stets immer ein Betrieb der Verdichter erforderlich ist, unabhängig davon, wie groß die von der Kühleinrichtung auszugleichende Temperaturdifferenz ist. Bei beispielsweise einer Kühlanforderung von >1 % einer Gesamtleistung muss bereits die volle Kälteleistung des betreffenden Verdichters bereitgestellt werden, um die angeforderte Soll-Temperatur am Wärmeübertrager halten zu können. Ein Großteil der Kälteleistung wird dann über den zuvor beschriebenen Bypass zum Verdichter zurückgeführt. Da ein fortwähren des ein- und ausschalten der Verdichter nicht möglich ist, und auch gegebenenfalls am Kondensator bzw. Gaskühler ein Lüfter betrieben werden muss, resultiert aus der hier beschriebenen bekannten Betriebsweise ein vergleichsweise hoher Energieverbrauch der Kühleinrichtung und eine verminderte Lebensdauer der Verdichter auch bei sehr geringen, auszugleichenden Temperaturdifferenzen.

Aus der US 2,434 221 ist ein Kühlkreislauf zur Temperierung eines Prüfraums mit einem Niederdruckverdichter und einem Hochdruckverdichter, einem Kondensator, einem Expansionsventil und einem Wärmeübertrager mit den Merkmalen des Oberbegriffs bekannt. Nachfolgend dem Kondensator ist eine Speichereinrichtung in dem Kühlkreislauf angeordnet, in dem gasförmiges und flüssiges Kältemittel gespeichert werden kann. Über einen Mitteldruckbypass mit einem Mitteldruckventil kann gasförmiges Kältemittel von der Speichereinrichtung auf eine Mitteldruckseite zwischen den Niederdruckverdichter und den Hochdruckverdichter geleitet werden.

Der vorliegenden Erfindung liegt daher die Aufgabe zugrunde, ein Verfahren zur Konditionierung von Luft in einem Prüfraum einer Prüfkammer sowie eine Prüfkammer vorzuschlagen, mit dem bzw. mit der die Prüfkammer wirtschaftlicher betrieben werden kann.

Diese Aufgabe wird durch ein Verfahren mit den Merkmalen des Anspruchs 1 und eine Prüfkammer mit den Merkmalen des Anspruchs 17 gelöst.

Bei dem erfindungsgemäßen Verfahren zur Konditionierung von Luft in einem gegenüber einer Umgebung verschließbaren und temperaturisolierten Prüfraum einer Prüfkammer zur Aufnahme von Prüfgut wird mittels einer Kühleinrichtung einer Temperiervorrichtung der Prüfkammer, mit einem Kühlkreislauf mit Kohlenstoffdioxid als einem Kältemittel, einem Wärmeübertrager im Prüfraum, einem Niederdruckverdichter und einem in einer Strömungsrichtung im Niederdruckverdichter nachfolgenden Hochdruckverdichter, einem Gaskühler, einer Speichereinrichtung für Kältemittel und einem Expansionsventil, eine Temperatur in einem Temperaturbereich von -20°C bis +180°C innerhalb des Prüfraums ausgebildet, wobei die Temperiervorrichtung eine Heizeinrichtung mit einer Heizung und einen Heiz-Wärmeübertrager in dem Prüfraum aufweist, wobei die Temperatur in dem Prüfraum mittels einer Steuervorrichtung der Prüfkammer gesteuert und/oder geregelt wird, wobei das Kältemittel über einen Bypass des Kühlkreislaufs an dem Niederdruckverdichter vorbei zu dem Hochdruckverdichter geleitet wird, wenn der Niederdruckverdichter abgeschaltet ist, wobei mittels eines nachfolgend im Gaskühler angeordneten Hochdruckventils des Kühlkreislaufs gasförmiges und/oder flüssiges Kältemittel in die Speichereinrichtung dosiert wird, wobei die Speichereinrichtung über einen Mitteldruckbypass des Kühlkreislaufs an einer Mitteldruckseite des Kühlkreislaufs in der Strömungsrichtung vor dem Hochdruckverdichter und nachfolgend dem Niederdruckverdichter angeschlossen ist, wobei mittels eines Mitteldruckventils des Kühlkreislaufs gasförmiges Kältemittel aus der Speichereinrichtung in die Mitteldruckseite dosiert wird, wenn der Niederdruckverdichter abgeschaltet ist.

Bei dem erfindungsgemäßen Verfahren wird durch eine Temperaturisolierung von Seitenwänden, Bodenwänden und Deckenwänden ein Wärmeaustausch mit einer Umgebung des Prüfraums weitgehend vermieden. Der Wärmeüberträger ist an den Kühlkreislauf angeschlossen bzw. in diesen so integriert, dass im Kühlkreislauf zirkulierendes Kältemittel durch den Wärmeübertrager strömt. Der Wärmeübertrager des Kühlreislaufs ist innerhalb des Prüfraums bzw. in einem Luftbehandlungsraum des Prüfraums angeordnet, so dass Luft in den Prüfraum über den Wärmeübertrager konditioniert bzw. temperiert wird. Der Gaskühler ist ebenso in den Kühlkreislauf integriert und als ein Wärmeübertrager ausgebildet. Der Gaskühler ist in dem Kühlkreislauf in Strömungsrichtung nachfolgend dem Hochdruckverdichter angeordnet, wobei das verdichtete Kältemittel, welches nach der Verdichtung unter einem hohen Druck steht und im Wesentlichen gasförmig vorliegt, in dem Gaskühler bzw. Kondensator kondensieren kann und dann im Wesentlichen in einem flüssigen Aggregatzustand vorliegt. Auch ist es möglich, dass das gasförmige Kältemittel in dem Gaskühler nicht kondensiert und den Gaskühler im Wesentlichen im gasförmigen Zustand wieder verlässt. Der Gaskühler bzw. der betreffende Wärmeübertrager kann mit Mitteln zur Kühlung des Kältemittels, beispielsweise durch Luft oder Wasser, ausgestattet sein. Das gasförmige und/oder flüssige Kältemittel strömt von dem Gaskühler über das Hochdruckventil in die Speichereinrichtung. Je nach Entnahmestelle an der Speichereinrichtung kann flüssiges oder gasförmiges Kältemittel aus der Speichereinrichtung entnommen werden. Das flüssige Kältemittel wird weiter über das Expansionsventil geleitet, wobei es durch Expansion in Folge eines Druckabfalls wiederum gasförmig wird. Dabei durchströmt es den Wärmeübertrager, der dadurch gekühlt wird. Nachfolgend wird das gasförmige Kältemittel wieder vom Niederdruckverdichter und/oder Hochdruckverdichter angesaugt und verdichtet.

Bei der vorliegenden Erfindung ist vorgesehen, dass das Hochdruckventil in Strömungsrichtung nachfolgend dem Gaskühler in dem Kühlkreislauf angeordnet ist, und gasförmiges und/oder flüssiges Kältemittel über das Hochdruckventil in die Speichereinrichtung dosiert wird. Die Speichereinrichtung ist im Wesentlichen ein Druckbehälter in dem bei Ausbildung einer Phasengrenze das flüssige Kältemittel in einem unteren Bereich und das gasförmige Kältemittel in einem oberen Bereich des Druckbehälters gespeichert ist. Aus der Speichereinrichtung wird dann je nach Entnahmestelle an der Speichereinrichtung flüssiges oder gasförmiges Kältemittel entnommen. So kann flüssiges Kältemittel dem Expansionsventil zugeführt und zur Kühlung des Wärmeübertragers in diesen entspannt, werden. Unter einem Expansionsventil wird zumindest ein Expansionsorgan, Drosselorgan, Drosselventil oder eine andere geeignete Verengung einer Fluidleitung verstanden. Das Expansionsventil bzw. das Mitteldruckventil und das Hochdruckventil sowie weitere Ventile des Kühlkreislaufs sind vorzugsweise regelbar ausgebildet.

Wenn nur eine sehr geringe Kälteleistung erforderlich ist, beispielsweise weniger als 2 % der Kälteleistung des Kühlkreislaufs, und/oder bei Temperaturen im Prüfraum von beispielsweise ≥-10 °C ist vorgesehen den Niederdruckverdichter abzuschalten. Unter einer Kälteleistung des Kühlkreislaufs wird hier eine thermische Energie bzw. eine Wärmeenergie oder auch ein Wärmeinhalt in Joule verstanden, wobei eine Zufuhr von Wärme eine thermische Energie steigert und eine Abfuhr von Wärme diese vermindert. Eine Abfuhr von Wärme am Wärmeübertrager über den Kühlkreislauf entspricht dann folglich einer entsprechenden Kälteleistung des Kühlkreislaufs. Da eine Leistung der Verdichter, beispielsweise bei Kompressoren, kaum regelbar ist, wird bei einer geringen benötigten Kälteleistung und/oder bei einer geringen Temperaturdifferenz zwischen einer Soll-Temperatur und einer Ist-Temperatur im Prüfraum der Niederdruckverdichter abgeschaltet und der Hochdruckverdichter weiter betrieben, um eine gegebenen falls erforderliche höhere Kälteleistung schnell zur Verfügung stellen zu können. Der Weiterbetrieb des Hochdruckverdichters wird dadurch ermöglicht, dass die Speichereinrichtung über den Mitteldruckbypass an die Mitteldruckseite des Kühlkreislaufs in der Strömungsrichtung vor dem Hochdruckverdichter und nachfolgend dem Niederdruckverdichter an die Mitteldruckseite angeschlossen ist. Der Mitteldruckbypass ist an einer Entnahmestelle an der Speichereinrichtung so angeschlossen, dass gasförmiges Kältemittel aus der Speichereinrichtung entnommen werden kann. Mittels des in dem Mitteldruckbypass angeordneten Mitteldruckventils wird nach Bedarf eine Dosierung des gasförmigen Kältemittels aus der Speichereinrichtung in die Mitteldruckseite ermöglicht. Der Hochdruckverdichter kann nun weiter betrieben werden, wenn keine oder nur eine sehr geringe Kälteleistung benötigt wird und/oder nur eine geringe Temperaturdifferenz im Prüfraum auszugleichen ist. So kann einerseits eine Betriebsbereitschaft durch ein Weiterbetreiben des Hochdruckverdichters hergestellt und andererseits der Niederdruckverdichter über einen längeren Zeitraum abgeschaltet werden sowie häufige Einschaltintervalle vermieden werden. Aufgrund einer so verringerten Gesamtlaufzeit der Verdichter und verringerter Schaltzyklen kann die Prüfkammer besonders energiesparend mit langer Lebensdauer betrieben werden.

Mittels der Temperiervorrichtung kann eine Temperatur in einem Temperaturbereich von -40 °C bis +180 °C, vorzugsweise -55 °C bis +180 °C innerhalb des Prüfraums ausgebildet werden. Sollen beispielsweise besonders niedrige Temperaturen > -10 °C in dem Prüfraum ausgebildet werden, kann vorgesehen sein den Hochdruckverdichter zusammen mit dem Niederdruckverdichter zu betreiben. In diesem Fall kann dann das Mitteldruckventil kurzzeitig geschlossen sein und über den Gaskühler kann Kältemittel verflüssigt und in die Speichereinrichtung eingeleitet werden. Grundsätzlich ist jedoch beabsichtigt das Mitteldruckventil offen zu halten, damit immer eine bestimmte Menge an gasförmigen Kältemittel in die Mitteldruckseite eingeleitet werden kann. Eine Eintrittstemperatur des Kältemittels am Gaskühler kann unterhalb des kritischen Punkts des Kältemittels liegen, sodass eine Verflüssigung des Kältemittels in dem Gaskühler erfolgt. Für den Fall, dass in der Speichereinrichtung zu wenig gasförmiges Kältemittel zur Leistungsregelung der Kühleinrichtung über den Mitteldruckbypass vorhanden ist, kann über das Hochdruckventil ein Druck kurzfristig unter einer Kondensationstemperatur des Kältemittels abgesenkt werden, sodass das Kältemittel nun nicht mehr im Gaskühler verflüssigt wird. Dieser Betriebszustand kann so lange beibehalten werden, bis wieder eine ausreichende Menge an gasförmigem Kältemittel in der Speichereinrichtung vorhanden ist.

Von der Steuervorrichtung kann bei einer Soll-Temperatur in einem Temperaturbereich von -10 °C bis +180 °C, vorzugsweise 0 °C bis +180 °C der Niederdruckverdichter abgeschaltet werden. Sofern in diesem Temperaturbereich eine besonders große Kälteleistung benötigt wird, kann auf einen Betrieb des Niederdruckverdichters verzichtet und Energie eingespart werden. In diesem Fall kann das Kältemittel beispielsweise über einen Bypass an dem Niederdruckverdichter vorbei zu dem Hochdruckverdichter geleitet werden.

Vorteilhaft kann der Kühlkreislauf in einem thermodynamisch unterkritischen, transkritischen oder überkritischen Betriebszustand betrieben werden. Je nach benötigter Kälteleistung bzw. je nachdem welche Temperaturdifferenz zwischen einer Ist-Temperatur und einer im Prüfraum zu erreichenden Soll-Temperatur vorliegt, kann der Kühlkreislauf in einem dieser Betriebszustände betrieben werden. Der Betriebszustand des Kühlkreislaufs ist prinzipiell von einer Eintrittstemperatur des Kühlmediums des Gaskühlers, zum Beispiel Kühlwasser oder Luft, am Gaskühler abhängig. Bei dem unterkritischen Betrieb des Kühlkreislaufs erfolgt eine Verflüssigung des Kältemittels im Gaskühler unterhalb des kritischen Punktes des Kältemittels sowie eine Entspannung am Expansionsventil und eine Umwandlung in die gasförmige Phase. Zumindest im unterkritischen Betriebszustand kann der Hochdruckverdichter und der Niederdruckverdichter betrieben werden. Der unterkritische Betrieb des Kühlkreislaufs entspricht einem Teillastbetrieb. Beim transkritischen und überkritischen Betriebszustand zirkuliert das Kältemittel im Kühlkreislauf im Wesentlichen gasförmigen Zustand. Das heißt eine Temperaturdifferenz ist soweit vermindert, dass das Kältemittel nicht im Gaskühler verflüssigt wird. Auch wird im transkritischen Betriebszustand ein Druck oberhalb des kritischen Punktes des Kältemittels am Gaskühler erreicht.

Im überkritischen Betriebszustand kann das Kältemittel am Hochdruckventil entspannt und als gasförmiges Kältemittel in die Speichereinrichtung geleitet werden kann. Der Gaskühler selbst kann beispielsweise mittels Luft oder Wasser gekühlt werden, sodass das Kältemittel ebenfalls beim Durchfluss durch den Gaskühler gekühlt wird. Der Gaskühler kann dann als ein Wärmeübertrager ausgebildet sein. Auch kann sichergestellt werden, dass das Kältemittel zumindest teilweise, vorzugsweise vollständig gasförmig aus dem Gaskühler austritt. Im transkritischen Betriebszustand kann hingegen ein Anteil an flüssigem Kältemittel vorliegen, wobei dann vorgesehen ist, dass dieser Anteil vergleichsweise groß ist. Das Kältemittel kann im transkritischen Betriebszustand auf der Hochdruckseite vollständig gasförmig sein, erst bei der Entspannung durch das Hochdruckventil kann dann ein Teil des Kältemittels flüssig werden. Das gasförmige und eventuell vorhandene flüssige Kältemittel wird über das Hochdruckventil in die Speichereinrichtung geleitet, wobei sich der Anteil an flüssigem Kältemittel in der Speichereinrichtung im transkritischen Betriebszustand sukzessive erhöhen kann.

Im transkritischen Betriebszustand kann mittels der Steuervorrichtung das Hochdruckventil derart geregelt werden, dass teilweise gasförmiges und flüssiges Kältemittel in die Speichereinrichtung geleitet wird. Demnach kann vorgesehen sein im transkritischen Betriebszustand über eine Regelung des Hochdruckventils einen Druck am Gaskühler soweit abzusenken oder eine Gasaustrittstemperatur am Gaskühler soweit anzuheben, dass das Kältemittel nicht mehr oder nur teilweise im Gaskühler verflüssigt wird. Das Kältemittel ist dann am Austritt des Gaskühlers noch gasförmig, bei der Entspannung kann es je nach Druck und Temperatur am Eintritt des Hochdruckventils mehr oder weniger flüssig sein.

Bei einer Entspannung des Kältemittels über das Hochdruckventil kann das Kältemittel gasförmig sein, wobei dann der transkritische Betriebszustand so lang aufrecht erhalten werden kann, wie gasförmiges Kältemittel in der Speichereinrichtung zur Verfügung steht und über den Mitteldruckbypass auf die Mitteldruckseite in Strömungsrichtung vor dem Hochdruckverdichter geleitet werden kann.

Demnach kann in Abhängigkeit einer Soll-Temperatur eine Leistung des Hochdruckverdichters angepasst werden, wobei im überkritischen Betriebszustand mittels der Steuervorrichtung das Mitteldruckventil derart geregelt werden kann, dass gasförmiges Kältemittel in die Mitteldruckseite geleitet wird. Das gasförmige Kältemittel kann dann von dem Hochdruckverdichter angesaugt und verdichtet werden, sodass der Hochdruckverdichter weiter betrieben werden kann obwohl keine oder nur eine geringe Kälteleistung benötigt wird. Prinzipiell kann bei allen Betriebszuständen zumindest ein Anteil an gasförmigen Kältemittel in die Mitteldruckseite geleitet werden. Ergänzend kann eine Anpassung der Leistung des Hochdruckverdichters auch noch dadurch erfolgen, dass eine Förderleistung des Hochdruckverdichters angepasst wird, beispielsweise durch einen Frequenzumrichter, wenn es sich bei dem Hochdruckverdichter um einen Kompressor handelt.

In dem Kühlkreislauf kann ein zweiter Mitteldruckbypass mit zumindest einem zweiten Mitteldruckventil ausgebildet sein, wobei der zweite Mitteldruckbypass in einer Strömungsrichtung nachfolgend der Speichereinrichtung und vor dem Expansionsventil sowie nachfolgend dem Niederdruckventil und vor dem Hochdruckverdichter an den Kühlkreislauf angeschlossen sein kann, wobei mittels des zweiten Mitteldruckventils flüssiges Kältemittel aus der Speichereinrichtung in die Mitteldruckseite dosiert werden kann. Dadurch ist es möglich das Kältemittel in dem Mitteldruckbereich abzukühlen. Wenn das Kältemittel bei der Kompression durch den Niederdruckverdichter erwärmt wird, kann so dem Hochdruckverdichter gekühltes Kältemittel zugeführt werden. Das zweite Mitteldruckventil kann beispielsweise als ein Einspritzventil ausgebildet sein, über das flüssiges Kältemittel in den Mitteldruckbereich eingespritzt werden kann. Der zweite Mitteldruckbypass kann derart an die Speichereinrichtung angeschlossen sein, dass ausschließlich flüssiges Kältemittel aus der Speichereinrichtung entnommen wird.

In dem Kühlkreislauf kann weiter ein Niederdruckbypass mit zumindest einem zweiten Expansionsventil ausgebildet sein, wobei der Niederdruckbypass in einer Strömungsrichtung nachfolgend der Speichereinrichtung und vor dem Expansionsventil sowie nachfolgend dem Wärmeübertrager und vor dem Niederverdichter an den Kühlkreislauf angeschlossen sein kann, wobei mittels des zweiten Expansionsventils flüssiges Kältemittel aus der Speichereinrichtung in eine Niederdruckseite dosiert werden kann. Eine Niederdruckseite des Kühlkreislaufs kann in Strömungsrichtung nachfolgend dem Expansionsventil und vor dem Niederdruckverdichter ausgebildet sein. Da sich der Wärmeübertrager im Prüfraum befindet kann bei besonders hohen Temperaturen im Prüfraum von beispielsweise +180 °C Kältemittel mit dieser Temperatur aus dem Wärmeübertrager zu dem Niederdruckverdichter und dem Hochdruckverdichter strömen. Bevor das stark erhitzte Kältemittel den Verdichtern zugeführt wird, kann es durch das über das zweite Expanionsventil dosierte Kältemittel abgekühlt werden.

In dem Kühlkreislauf kann ein Regelbypass mit zumindest einem Regelventil ausgebildet sein, wobei der Regelbypass in einer Strömungsrichtung nachfolgend dem Wärmeübertrager und vor dem Niederdruckverdichter sowie nachfolgend dem Niederdruckverdichter und vor dem Hochdruckverdichter an den Kühlkreislauf angeschlossen sein kann, wobei mittels des Regelventils gasförmiges Kältemittel aus der Mitteldruckseite in eine Niederdruckseite dosiert werden kann, wobei der Niederdruckverdichter betrieben werden kann. Beispielsweise kann der Niederdruckverdichter zusammen mit dem Hochdruckverdichter in einem Temperaturbereich von ≤-10 °C betrieben werden, wobei das Regelventil dann zunächst vollständig geschlossen werden kann. Das Regelventil kann dann zu einer Leistungsregelung des Niederdruckverdichters genutzt werden, indem kaltes gasförmiges Kältemittel von der Mitteldruckseite zurück auf die Niederdruckseite geleitet wird. In diesem Fall kann dann auch auf eine Einspritzung von flüssigem Kältemittel in die Niederdruckseite, beispielsweise über einen Niederdruckbypass, verzichtet werden.

Von der Steuervorrichtung kann mittels des Regelbypasses ein Saugdruck vor dem Niederdruckverdichter eingestellt werden, derart, dass sich das Kältemittel vor dem Niederdruckverdichter in einem Zustand unterhalb des Tripelpunktes befindet. Durch einen Verzicht einer Einspritzung von flüssigem Kältemittel in die Niederdruckseite und die Zuführung von kaltem, gasförmigen Kältemittel von der Mitteldruckseite kann ein Saugdruck vor dem Niederdruckverdichter unterhalb des Tripelpunktes abgesenkt werden ohne dass es hier zu einer Trockeneisbildung kommt. Dies ist besonders vorteilhaft bei langen Saugleitungen, um Druckverluste über die Saugleitung zu kompensieren und um sicherzustellen, dass bei tiefen Temperaturen im Prüfraum eine ausreichend hohe Differenz zwischen der Temperatur im Prüfraum und einer Temperatur des Wärmeübertragers bzw. eine Verdampfungstemperatur des Kältemittels vorhanden ist.

In dem Kühlkreislauf kann ein Regelbypass mit zumindest einem Regelventil ausgebildet sein, wobei der Regelbypass in einer Strömungsrichtung nachfolgend dem Wärmeübertrager und vor dem Niederdruckverdichter sowie nachfolgend dem Niederdruckverdichter und vor dem Hochdruckverdichter an dem Kühlkreislauf angeschlossen sein kann, wobei mittels des Regelventils gasförmiges Kältemittel aus einer Niederdruckseite in die Mitteldruckseite dosiert werden kann, wobei der Niederdruckverdichter dann abgeschaltet sein kann. So kann es ausreichend sein bei einem Temperaturbereich von ≥ -10 °C im Prüfraum alleine über den Hochdruckverdichter eine Kälteleistung zur Verfügung zu stellen und den Niederdruckverdichter abzuschalten. Dann kann das Regelventil vollständig geöffnet werden, sodass dann die Niederdruckseite und die Mitteldruckseite miteinander verbunden sind. Ein Massenstrom des Kältemittels kann an dem Niederdruckverdichter vorbei zu dem Hochdruckverdichter geführt werden. Das Regelventil kann auch zur Regelung eines Drucks auf der Niederdruckseite genutzt werden. Demnach kann der Regelbypass mit dem Regelventil bei verschiedenen Betriebszuständen der Kühleinrichtung verwendet werden, nämlich in einem Temperaturbereich von -10 °C bis -55°C durch Entnahme von kalten gasförmigen Kältemittel aus der Mitteldruckseite und Einleitung in die Niederdruckseite vor dem Niederdruckverdichter, wobei ein niedriger Saugdruck unterhalb des Tripelpunktes möglich ist, oder zur Druckregelung auf der Niederdruckseite bzw. im Wärmeübertrager, wenn der Niederdruckverdichter in einem Klimabetrieb der Kühleinrichtung abgeschaltet ist, und/oder zur Leistungsregelung des Niederdruckverdichters, wenn der Niederdruckverdichter und der Hochdruckverdichter gleichzeitig betrieben werden. Eine Regelung eines Saugdrucks des Hochdruckverdichters kann über das Mitteldruckventil erfolgen.

Auch kann die Temperiervorrichtung einen Entfeuchter aufweisen, der aus einem Entfeuchterbypass mit einem Entfeuchterventil und einem weiteren Wärmeübertrager im Prüfraum ausgebildet ist, wobei der Entfeuchterbypass an der Speichereinrichtung sowie nachfolgend dem Niederdruckverdichter und vor dem Hochdruckverdichter an der Mitteldruckseite angeschlossen sein kann, wobei mittels des Entfeuchterventils Kältemittel aus der Speichereinrichtung in die Mitteldruckseite dosiert werden kann, wenn der Niederdruckverdichter abgeschaltet ist oder betrieben wird. Der weitere Wärmeübertrager kann dabei einen sogenannten Nassentfeuchter ausbilden, der ebenfalls im Prüfraum angeordnet ist, wobei dann regelmäßig der Wärmeübertrager gegenüber dem Wärmeübertrager eine geringere Temperatur aufweisen sollte, sodass eine Kondensation an dem weiteren Wärmeübertrager und nicht an dem Wärmeübertrager erfolgt. Das Entfeuchterventil kann ein Expansionsventil sein, über das flüssiges Kältemittel in den weiteren Wärmeübertrager entspannt wird. Das aus dem weiteren Wärmeübertrager austretende Kältemittel kann im Wesentlichen gasförmig sein und gelangt nachfolgend dem Niederdruckverdichter in die Mitteldruckseite. Durch den Anschluss des Entfeuchterbypasses an der Mitteldruckseite kann der Hochdruckverdichter noch besser ausgelastet werden, wenn der Niederdruckverdichter abgeschaltet ist. Wird der Niederdruckverdichter betrieben, ist es vorteilhaft, wenn in dem Entfeuchterbypass ein Rückschlagventil angeordnet ist, welches ein Zurückfließen von Kältemittel von der Mitteldruckseite in den weiteren Wärmeübertrager verhindert. Je nach vorliegendem Sättigungsdampfruck der Mitteldruckseite könnte sonst gasförmiges Kältemittel im weiteren Wärmeübertrager kondensieren.

Von der Steuervorrichtung kann mittels Steuerung des Expansionsventils und/oder des Entfeuchterventils eine Verdampfungstemperatur an dem weiteren Wärmeübertrager niedriger als eine Verdampfungstemperatur an dem Wärmeübertrager ausgebildet werden. So kann in jedem Fall sichergestellt werden, dass die im Prüfraum befindliche Luft bzw. das darin enthaltene Wasser an dem weiteren Wärmeübertrager kondensiert bzw. an dem Wärmeübertrager kondensiertes Wasser nicht gefrieren kann. Dabei kann vorgesehen sein, dass die Steuervorrichtung ein Regelventil eines Regelbypasses des Kühlkreislaufs ergänzend steuert.

Besonders vorteilhaft ist es, wenn reines Kohlenstoffdioxid als das Kältemittel verwendet wird. Reines Kohlenstoffdioxid weist einen GWP von 1 auf, ist nicht brennbar, ungefährlich und kostengünstig erhältlich. Darüber hinaus ist Kohlenstoffdioxid ein Reinstoff bzw. azeotrop, was die vorteilhafte Durchführung des Verfahrens und dessen Varianten überhaupt erst ermöglicht. Ein Kältemittel mit zeotropem Verhalten würde hingegen eine Bereitstellung einer ausreichenden Menge von gasförmigen Kältemittel bei einer sehr geringen Temperaturdifferenz kaum ermöglichen und somit eine Leistungsregelung des Hochdruckverdichters kaum zulassen.

Die erfindungsgemäße Prüfkammer, insbesondere Klimakammer zur Konditionierung von Luft, umfasst einen gegenüber einer Umgebung verschließbaren und temperaturgedämmten Prüfraum zur Aufnahme von Prüfgut, und eine Temperiervorrichtung zur Temperierung des Prüfraums, wobei mittels der Temperiervorrichtung eine Temperatur in einem Temperaturbereich von -20 °C bis +180 °C innerhalb des Prüfraums ausbildbar ist, wobei die Temperiervorrichtung eine Heizeinrichtung mit einer Heizung und einen Heiz-Wärmeübertrager in dem Prüfraum aufweist, wobei die Temperiervorrichtung eine Kühleinrichtung mit einem Kühlkreislauf mit Kohlenstoffdioxid als einem Kältemittel, einem Wärmeübertrager im Prüfraum, einem Niederdruckverdichter und einem in einer Strömungsrichtung dem Niederdruckverdichter nachfolgenden Hochdruckverdichter, einem Gaskühler, einer Speichereinrichtung für Kältemittel und einem Expansionsventil aufweist, wobei die Prüfkammer eine Steuervorrichtung zur Steuerung und/oder Regelung der Temperatur in dem Prüfraum aufweist, wobei das Kältemittel über einen Bypass des Kühlkreislaufs an dem Niederdruckverdichter vorbei zu dem Hochdruckverdichter leitbar ist, wenn der Niederdruckverdichter abgeschaltet ist, wobei der Kühlkreislauf in der Strömungsrichtung nachfolgend dem Gaskühler ein Hochdruckventil aufweist, mittels dem gasförmiges und/oder flüssiges Kältemittel in die Speichereinrichtung dosierbar ist, wobei die Speichereinrichtung über einen Mitteldruckbypass des Kühlkreislaufs an einer Mitteldruckseite des Kühlkreislaufs in der Strömungsrichtung vor dem Hochdruckverdichter und nachfolgend dem Niederdruckverdichter angeschlossen ist, wobei mittels eines Mitteldruckventils des Kühlkreislaufs gasförmiges Kältemittel aus der Speichereinrichtung in die Mitteldruckseite dosierbar ist, wobei die Steuervorrichtung dazu eingerichtet ist, mittels des Mitteldruckventils gasförmiges Kältemittel in die Mitteldruckseite zu dosieren, wenn der Niederdruckverdichter abgeschaltet ist. Zu den Vorteilen der erfindungsgemäßen Prüfkammer wird auf die Vorteilsbeschreibung des erfindungsgemäßen Verfahrens verwiesen.

Die Temperiervorrichtung weist erfindungsgemäß eine Heizeinrichtung mit einer Heizung und einem Heiz-Wärmeübertrager in dem Prüfraum auf. Die Heizeinrichtung kann beispielsweise eine elektrische Widerstandsheizung sein, die den Heiz-Wärmeübertrager beheizt, derart, dass über den Heiz-Wärmeübertrager eine Temperaturerhöhung im Prüfraum ermöglicht wird. Wenn der Wärmeübertrager und der Heiz-Wärmeübertrager mittels der Steuervorrichtung zur Kühlung oder Erwärmung der im Prüfraum umgewälzten Luft gezielt gesteuert bzw. geregelt werden können, kann mittels der Temperiervorrichtung dann innerhalb des Prüfraums eine Temperatur in den vorstehend angegebenen Temperaturbereichen ausgebildet werden.

Weitere Ausführungsformen einer Prüfkammer ergeben sich aus den Merkmalsbeschreibungen der auf den Verfahrensanspruch 1 rückbezogenen Unteransprüche.

Nachfolgend wird die Erfindung in bevorzugter Ausführungsform der Erfindung unter Bezugnahme auf die beigefügten Zeichnungen näher erläutert.

Es zeigen:
- **Fig. 1**: Eine schematische Darstellung einer Ausführungsform einer Kühleinrichtung;
- **Fig. 2**: ein Druck-Enthalpie-Diagramm mit einem ersten Betriebszustand eines Kühlkreislaufs;
- **Fig. 3**: das Druck-Enthalpie-Diagramm mit einem zweiten Betriebszustand des Kühlkreislaufs;
- **Fig. 4**: das Druck-Enthalpie-Diagramm mit einem dritten Betriebszustand des Kühlkreislaufs;
- **Fig. 5**: das Druck-Enthalpie-Diagramm mit einem vierten Betriebszustand des Kühlkreislaufs.

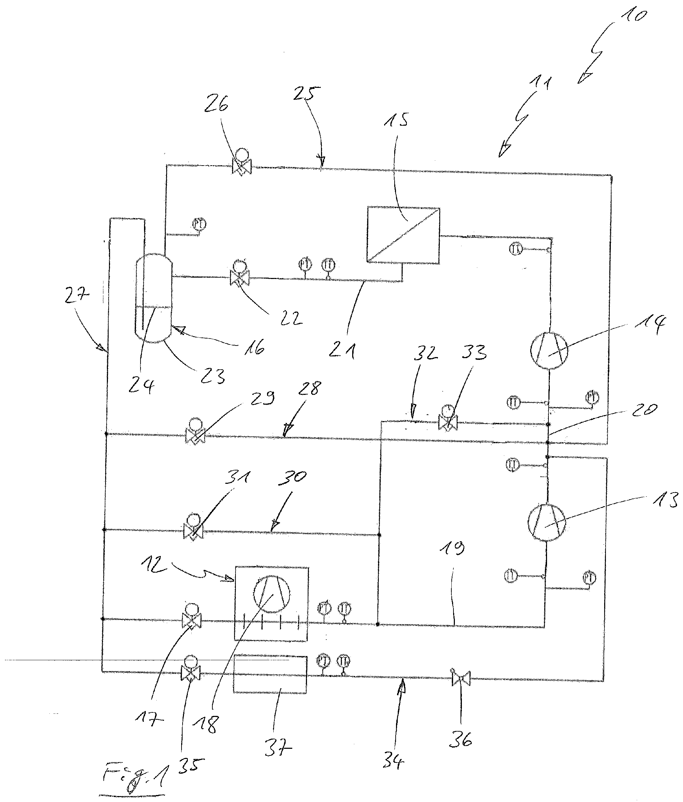

Die **Fig. 1** zeigt eine mögliche Ausführungsform einer Kühleinrichtung 10 einer hier nicht dargestellten Prüfkammer. Die Kühleinrichtung 10 umfasst einen Kühlkreislauf 11 mit Kohlenstoffdioxid (CO₂) als einem Kältemittel, einen Wärmeübertrager 12, einen Niederdruckverdichter 13, einen Hochdruckverdichter 14, einen Gaskühler 15, eine Speichereinrichtung 16, und ein Expansionsventil 17. Der Gaskühler 15 ist hier in Art eines Wärmeübertragers ausgebildet über ein Wärmeträgermedium, wie beispielsweise Luft oder Wasser, gekühlt. Der Wärmeübertrager 12 ist in einem hier nicht dargestellten Luftbehandlungskanal der Prüfkammer angeordnet, derart, dass ein Lüfter 18 die Luft im Prüfraum an dem Wärmeübertrager 12 umwälzen kann. Weiter weist der Kühlkreislauf 11 eine Niederdrucksseite 19, eine Mitteldruckseite 20 und eine Hochdruckseite 21 auf. In der Niederdruckseite 19 ist ein Druck des Kältemittels vergleichsweise niedriger als in der Mitteldruckseite 20. In der Mitteldruckseite 20 ist ein Druck des Kältemittels vergleichsweise niedriger als in der Hochdruckseite 21.

Der Kühlkreislauf 11 weist weiter in einer Strömungsrichtung des Kältemittels nachfolgend dem Gaskühler 15 ein Hochdruckventil 22 auf, über das gasförmiges und/oder flüssiges Kältemittel in die Speichereinrichtung 16 entspannt bzw. dosiert wird. Die Speichereinrichtung 16 ist als ein Druckbehälter 23 ausgebildet, in dem sich eine Phasengrenze 24 zwischen dem flüssigen und dem gasförmigen Kältemittel ausbildet. An der Speichereinrichtung 16 ist ein Mitteldruckbypass 25 mit einem Mitteldruckventil 26 des Kühlkreislaufs 11 so angeschlossen, dass aus der Speichereinrichtung 16 gasförmiges Kältemittel entnommen und auf die Mitteldruckseite 20 in Strömungsrichtung des Kältemittels nachfolgend dem Niederdruckverdichter 13 und vor dem Hochdruckverdichter 14 geleitet werden kann. Weiter ist an der Speichereinrichtung 16 ein Leitungsabschnitt 27 so angeschlossen, dass aus der Speichereinrichtung 16 flüssiges Kältemittel entnommen und zu dem Expansionsventil 17 geleitet werden kann.

Darüber hinaus umfasst der Kühlkreislauf 11 einen zweiten Mitteldruckbypass 28 mit einem zweiten Mitteldruckventil 29, wobei der zweite Mitteldruckbypass 28 in einer Strömungsrichtung nachfolgend der Speichereinrichtung 16 an dem Leitungsabschnitt 27 sowie nachfolgend dem Niederdruckverdichter 13 und vor dem Hochdruckverdichter 14 an den Kühlkreislauf 11 angeschlossen ist. Mittels des zweiten Mitteldruckventils 29 kann flüssiges Kältemittel aus der Speichereinrichtung 16 in die Mitteldruckseite 20 dosiert werden.

Weiter umfasst der Kühlkreislauf 11 einen Niederdruckbypass 30 mit einem zweiten Expansionsventil 31, wobei der Niederdruckbypass 30 in einer Strömungsrichtung nachfolgend der Speichereinrichtung 16 am Leitungsabschnitt 27 sowie nachfolgend dem Wärmeübertrager 12 und vor dem Niederdruckverdichter 13 an der Niederdruckseite 19 des Kühlkreislaufs 11 angeschlossen ist. Mittel des zweiten Expansionsventils 31 kann flüssiges Kältemittel aus der Speichereinrichtung 16 in die Niederdruckseite 19 dosiert werden.

In dem Kühlkreislauf 11 ist weiter ein Regelbypass 32 mit einem Regelventil 33 ausgebildet, wobei der Regelbypass 32 in einer Strömungsrichtung des Kältemittels nachfolgend dem Wärmeübertrager 12 und vor dem Niederdruckverdichter 13 an der Niederdruckseite 19 sowie nachfolgend dem Niederdruckverdichter 13 und vor dem Hochdruckverdichter 14 an der Mitteldruckseite 20 angeschlossen ist. Mit dem Regelventil 33 kann in Abhängigkeit eines Betriebszustandes des Kühlkreislaufes 11 Kältemittel von der Niederdruckseite 19 in die Mitteldruckseite 20 oder umgekehrt, von der Mitteldruckseite 20 in die Niederdruckseite 19, geleitet werden.

Zur Entfeuchtung des Prüfraums weist der Kühlkreislauf 11 einen Entfeuchterbypass 34 mit einem Entfeuchterventil 35, einem Rückschlagventil 36 und einen weiteren Wärmeübertrager 37 auf. Der weitere Wärmeübertrager 37 ist in dem hier nicht dargestellten Prüfraum angeordnet. Der Entfeuchterbypass 34 ist an der Speichereinrichtung 16 über den Leitungsabschnitt 27 sowie nachfolgend dem Niederdruckverdichter 13 und vor dem Hochdruckverdichter 14 an der Mitteldruckseite 20 angeschlossen. Mittels des Entfeuchterventils 35 kann flüssiges Kältemittel aus der Speichereinrichtung 16 in den weiteren Wärmeübertrager 37 expandiert und in die Mitteldruckseite 20 eingeleitet werden. Der weitere Wärmeübertrager 37 wird dabei so abgekühlt, dass das in der im Prüfraum befindlichen Luft enthaltene Wasser im Wesentlichen an dem weiteren Wärmeübertrager 37 kondensiert bzw. nicht am Wärmeübertrager 12 gefriert. Das Rückschlagventil 36 verhindert einen Rückfluss des Kältemittels in den weiteren Wärmeübertrager 37 aus der Mitteldruckseite 20 wenn sich dort aufgrund einer Druckerhöhung, beispielsweise durch den Betrieb des Niederdruckverdichters 13, ein Druckgefälle gegenüber dem weiteren Wärmeübertrager 37 einstellt.

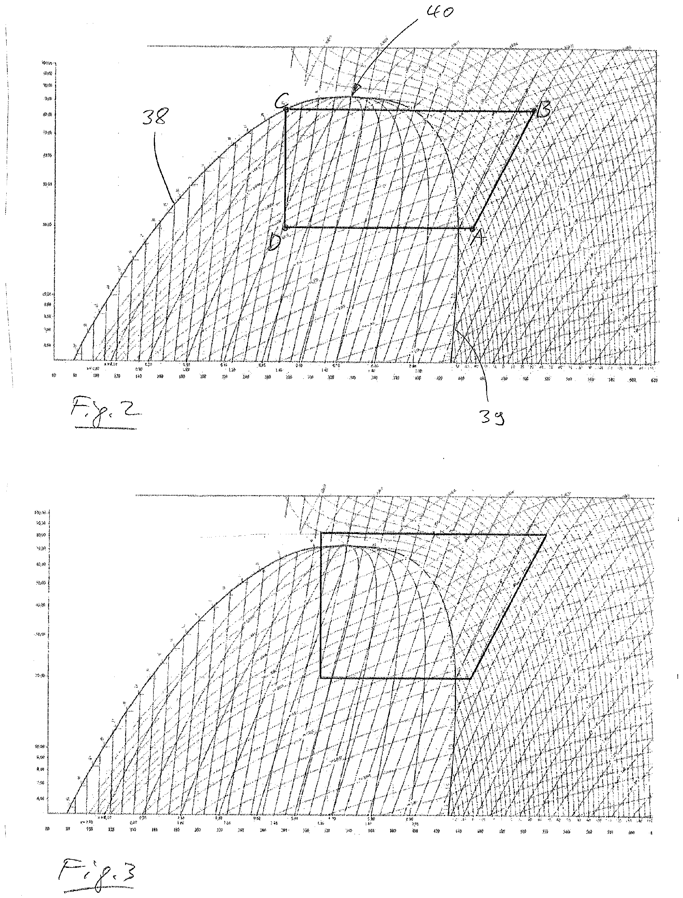

Die **Fig. 2** bis **5** zeigen jeweils Druck-Enthalpie-Diagramme (Log-p-h-Diagramm) für das im Kühlkreislauf 11 zirkulierende Kältemittel in unterschiedlichen Betriebszuständen des Kühlkreislaufs 11 bei alleinigem Betrieb des Hochdruckverdichters 14. In den jeweiligen Diagrammen ist die spezifische Enthalpie auf der Abszissenachse und der logarithmisch skalierte Druck auf der Ordinatenachse dargestellt. Eine Siedelinie 38 markiert einen Übergang von gesättigter Flüssigkeit im Nassdampf, wobei eine Taulinie 39 einen Übergang von Nassdampf zu gesättigtem Dampf markiert. Siedelinie 38 und Taulinie 39 treffen sich im kritischen Punkt 40.

Die **Fig. 2** zeigt einen unterkritischen Betriebszustand des Kühlkreislaufs 11, bei dem ausgehend von der Position A das Kältemittel von der Niederdruckseite 19 mit dem Hochdruckverdichter 14 angesaugt und komprimiert wird, sodass ein Druck entsprechend der Position B in Strömungsrichtung nach dem Verdichter 14 erzielt wird. Das Kältemittel wird nachfolgend in dem Gaskühler 15 entsprechend der Position C verflüssigt und über das Hochdruckventil 22 in die Speichereinrichtung 16 geleitet. Im Expansionsventil 17 erfolgt eine Entspannung des Kältemittels (Positionen C bis D), wobei das Kältemittel in dem Wärmeübertrager 12 verdampft (Positionen D bis A).

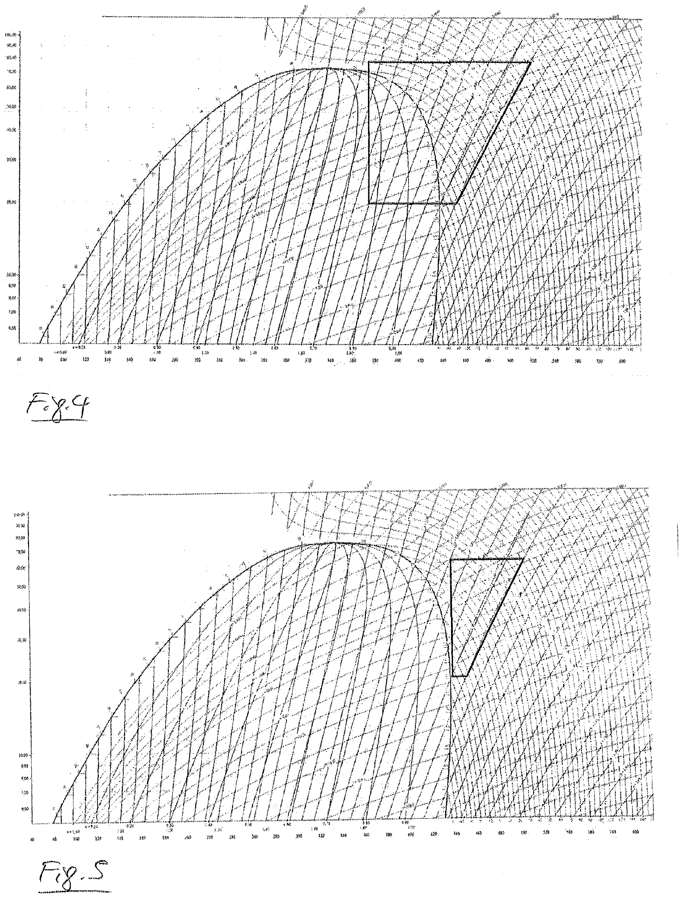

Die **Fig. 3** und **4** zeigen transkritische Betriebszustände und die **Fig. 5** einen überkritischen Betriebszustand des Kühlkreislaufs 11 bei alleinigem Betrieb des Hochdruckverdichters 14.

Der überkritische Betriebszustand wird dadurch erzielt, dass der Niederdruckverdichter 13 abgeschaltet wird, wenn keine oder eine sehr geringe Kälteleistung am Wärmeübertrager 12 abgegeben werden muss und/oder eine Temperatur im Prüfraum von ≥ -10°C erreicht werden soll. Der überkritische Betriebszustand wird dann zur Herstellung von gasförmigen Kältemittel genutzt. Dazu wird ein Druck in der Mitteldruckseite 20 angehoben, in dem das Mitteldruckventil 26 geöffnet und gasförmiges Kältemittel über den Mitteldruckbypass 35 auf die Mitteldruckseite 20 geleitet wird. Ergänzend dazu kann auch eine Förderleistung des Hochdruckverdichters 14 über einen Frequenzumrichter zunächst abgesenkt werden. Wird keinerlei Kälteleistung benötigt, sind das Expansionsventil 17 und das Entfeuchterventil 35 vollständig geschlossen, wobei dann das Kältemittel alleine über den Mitteldruckbypass 25 zirkuliert. Um in dem überkritischen Betriebszustand genügend gasförmiges Kältemittel zur Verfügung zu stellen, wird das Hochdruckventil 22 weiter geöffnet, sodass durch das Absenken des Drucks in der Hochdruckseite 21 bei einer Entspannung des Kältemittels mehr Dampf anfällt, welcher zur Leistungsanpassung des Hochdruckverdichters 14 genutzt werden kann. Auch kann eine Austrittstemperatur des Kältemittels am Gaskühler 15 angehoben und das Hochdruckventil 22 vollständig geöffnet werden, sodass bei der Entspannung des Kältemittels keine Flüssigkeit entsteht und ausschließlich gasförmiges Kältemittel zirkuliert wird. Insgesamt ist es so möglich auf ein Abschalten des Hochdruckverdichters 14 zu verzichten und diesen weiter zu betreiben, selbst wenn keine oder nur eine sehr geringe Kälteleistung erbracht werden muss.

## Patentansprüche

1. Verfahren zur Konditionierung von Luft in einem gegenüber einer Umgebung verschließbaren und temperaturgedämmten Prüfraum einer Prüfkammer zur Aufnahme von Prüfgut, wobei mittels einer Kühleinrichtung (10) einer Temperiervorrichtung der Prüfkammer, mit einem Kühlkreislauf (11) mit einem Kältemittel, einem Wärmeübertrager (12) im Prüfraum, einem Niederdruckverdichter (13) und einem in einer Strömungsrichtung dem Niederdruckverdichter nachfolgenden Hochdruckverdichter (14), einem Gaskühler (15), einer Speichereinrichtung (16) für Kältemittel und einem Expansionsventil (17) eine Temperatur innerhalb des Prüfraums ausgebildet wird, wobei die Temperatur in dem Prüfraum mittels einer Steuervorrichtung der Prüfkammer gesteuert und/oder geregelt wird, wobei das Kältemittel über einen Bypass des Kühlkreislaufs an dem Niederdruckverdichter vorbei zu dem Hochdruckverdichter geleitet wird, wenn der Niederdruckverdichter abgeschaltet ist, wobei mittels eines in einer Strömungsrichtung nachfolgend dem Gaskühler angeordneten Hochdruckventils (22) des Kühlkreislaufs gasförmiges und/oder flüssiges Kältemittel in die Speichereinrichtung dosiert wird, wobei die Speichereinrichtung über einen Mitteldruckbypass (25) des Kühlkreislaufs an einer Mitteldruckseite (20) des Kühlkreislaufs in der Strömungsrichtung vor dem Hochdruckverdichter und nachfolgend dem Niederdruckverdichter angeschlossen ist,
**dadurch gekennzeichnet,**
**dass** das Kältemittel Kohlenstoffdioxid (CO₂) ist, wobei die Temperatur in einem Temperaturbereich von -20 °C bis +180 °C ausgebildet wird, wobei die Temperiervorrichtung eine Heizeinrichtung mit einer Heizung und einen Heiz-Wärmeübertrager in dem Prüfraum aufweist, wobei mittels eines Mitteldruckventils (26) des Kühlkreislaufs gasförmiges Kältemittel aus der Speichereinrichtung in die Mitteldruckseite dosiert wird, wenn der Niederdruckverdichter abgeschaltet ist.

2. Verfahren nach Anspruch 1,
**dadurch gekennzeichnet,**
**dass** mittels der Temperiervorrichtung eine Temperatur in einem Temperaturbereich von -40 °C bis +180 °C, vorzugsweise -55 °C bis +180 °C, innerhalb des Prüfraums ausgebildet wird.

3. Verfahren nach Anspruch 1 oder 2,
**dadurch gekennzeichnet,**
**dass** von der Steuervorrichtung bei einer Soll-Temperatur in einem Temperaturbereich von -10 °C bis +180 °C, vorzugsweise 0 °C bis +180 °C der Niederdruckverdichter (13) abgeschaltet wird.

4. Verfahren nach einem der vorangehenden Ansprüche,
**dadurch gekennzeichnet,**
**dass** der Kühlkreislauf (11) in einem thermodynamisch unterkritischen, transkritischen oder überkritischen Betriebszustand betrieben wird.

5. Verfahren nach Anspruch 4,
**dadurch gekennzeichnet,**
**dass** zumindest im unterkritischen Betriebszustand der Hochdruckverdichter (14) und der Niederdruckverdichter (13) betrieben werden.

6. Verfahren nach Anspruch 4 oder 5,
**dadurch gekennzeichnet,**
**dass** im überkritischen Betriebszustand das Kältemittel am Hochdruckventil (22) entspannt und als gasförmiges Kältemittel in die Speichereinrichtung (16) geleitet wird.

7. Verfahren nach einem der Ansprüche 4 bis 6,
**dadurch gekennzeichnet,**
**dass** im transkritischen Betriebszustand mittels der Steuervorrichtung das Hochdruckventil (22) derart geregelt wird, dass teilweise gasförmiges und flüssiges Kältemittel in die Speichereinrichtung (16) geleitet wird.

8. Verfahren nach einem der Ansprüche 4 bis 7,
**dadurch gekennzeichnet,**
**dass** in Abhängigkeit einer Soll-Temperatur eine Leistung des Hochdruckverdichters (14) angepasst wird, wobei im überkritischen Betriebszustand mittels der Steuervorrichtung das Mitteldruckventil (26) derart geregelt wird, dass gasförmiges Kältemittel in die Mitteldruckseite (20) geleitet wird.

9. Verfahren nach einem der vorangehenden Ansprüche,
**dadurch gekennzeichnet,**
**dass** in dem Kühlkreislauf (11) ein zweiter Mitteldruckbypass (28) mit zumindest einem zweiten Mitteldruckventil (29) ausgebildet ist, wobei der zweite Mitteldruckbypass in einer Strömungsrichtung nachfolgend der Speichereinrichtung (16) und vor dem Expansionsventil (17) sowie nachfolgend dem Niederdruckverdichter (13) und vor dem Hochdruckverdichter (14) an den Kühlkreislauf angeschlossen ist, wobei mittels des zweiten Mitteldruckventils flüssiges Kältemittel aus der Speichereinrichtung in die Mitteldruckseite (20) dosiert wird.

10. Verfahren nach einem der vorangehenden Ansprüche,
**dadurch gekennzeichnet,**
**dass** in dem Kühlkreislauf (11) ein Niederdruckbypass (30) mit zumindest einem zweiten Expansionsventil (31) ausgebildet ist, wobei der Niederdruckbypass in einer Strömungsrichtung nachfolgend der Speichereinrichtung (16) und vor dem Expansionsventil (17) sowie nachfolgend dem Wärmeübertrager (12) und vor dem Niederdruckverdichter (13) an den Kühlkreislauf angeschlossen ist, wobei mittels des zweiten Expansionsventils flüssiges Kältemittel aus der Speichereinrichtung in eine Niederdruckseite (19) dosiert wird.

11. Verfahren nach einem der vorangehenden Ansprüche,
**dadurch gekennzeichnet,**
**dass** in dem Kühlkreislauf (11) ein Regelbypass (32) mit zumindest einem Regelventil (33) ausgebildet ist, wobei der Regelbypass in einer Strömungsrichtung nachfolgend dem Wärmeübertrager (12) und vor dem Niederdruckverdichter (13) sowie nachfolgend dem Niederdruckverdichter und vor dem Hochdruckverdichter (14) an den Kühlkreislauf angeschlossen ist, wobei mittels des Regelventils gasförmiges Kältemittel aus der Mitteldruckseite (20) in eine Niederdruckseite (19) dosiert wird, wobei der Niederdruckverdichter betrieben wird.

12. Verfahren nach Anspruch 11,
**dadurch gekennzeichnet,**
**dass** von der Steuervorrichtung mittels des Regelbypasses (32) ein Saugdruck vor dem Niederdruckverdichter (13) eingestellt wird, derart, dass sich das Kältemittel vor dem Niederdruckverdichter in einem Zustand unterhalb des Tripelpunktes befindet.

13. Verfahren nach einem der Ansprüche 1 bis 10,
**dadurch gekennzeichnet,**
**dass** in dem Kühlkreislauf (11) ein Regelbypass (32) mit zumindest einem Regelventil (33) ausgebildet ist, wobei der Regelbypass in einer Strömungsrichtung nachfolgend dem Wärmeübertrager (12) und vor dem Niederdruckverdichter (13) sowie nachfolgend dem Niederdruckverdichter und vor dem Hochdruckverdichter (14) an den Kühlkreislauf angeschlossen ist, wobei mittels des Regelventils gasförmiges Kältemittel aus einer Niederdruckseite (19) in die Mitteldruckseite (20) dosiert wird, wobei der Niederdruckverdichter abgeschaltet ist.

14. Verfahren nach einem der vorangehenden Ansprüche,
**dadurch gekennzeichnet,**
**dass** die Temperiervorrichtung einen Entfeuchter aufweist, der aus einem Entfeuchterbypass (34) mit einem Entfeuchterventil (35) und einem weiteren Wärmeübertrager (37) im Prüfraum ausgebildet ist, wobei der Entfeuchterbypass an der Speichereinrichtung (16) sowie nachfolgend dem Niederdruckverdichter (13) und vor dem Hochdruckverdichter (14) an der Mitteldruckseite (20) angeschlossen ist, wobei mittels des Entfeuchterventils Kältemittel aus der Speichereinrichtung in die Mitteldruckseite dosiert wird, wenn der Niederdruckverdichter abgeschaltet ist oder betrieben wird.

15. Verfahren nach Anspruch 14,
**dadurch gekennzeichnet,**
**dass** von der Steuervorrichtung mittels Steuerung des Expansionsventils (17) und/oder des Entfeuchterventils (35) eine Verdampfungstemperatur an dem weiteren Wärmeübertrager (37) niedriger als eine Verdampfungstemperatur an dem Wärmeübertrager (12) ausgebildet wird.

16. Verfahren nach einem der vorangehenden Ansprüche,
**dadurch gekennzeichnet,**
**dass** reines Kohlenstoffdioxid (CO₂) als das Kältemittel verwendet wird.

17. Prüfkammer, insbesondere Klimakammer zur Konditionierung von Luft, umfassend einen gegenüber einer Umgebung verschließbaren und temperaturgedämmten Prüfraum zur Aufnahme von Prüfgut, und eine Temperiervorrichtung zur Temperierung des Prüfraums, wobei mittels der Temperiervorrichtung eine Temperatur innerhalb des Prüfraums ausbildbar ist, wobei die Temperiervorrichtung eine Kühleinrichtung (10) mit einem Kühlkreislauf (11) mit einem Kältemittel, einem Wärmeübertrager (12) im Prüfraum, einem Niederdruckverdichter (13) und einem in einer Strömungsrichtung dem Niederdruckverdichter nachfolgenden Hochdruckverdichter (14), einem Gaskühler (15), einer Speichereinrichtung (16) für Kältemittel und einem Expansionsventil (17) aufweist, wobei die Prüfkammer eine Steuervorrichtung zur Steuerung und/oder Regelung der Temperatur in dem Prüfraum aufweist, wobei das Kältemittel über einen Bypass des Kühlkreislaufs an dem Niederdruckverdichter vorbei zu dem Hochdruckverdichter leitbar ist, wenn der Niederdruckverdichter abgeschaltet ist, wobei der Kühlkreislauf in einer Strömungsrichtung nachfolgend dem Gaskühler ein Hochdruckventil (22) aufweist, mittels dem gasförmiges und/oder flüssiges Kältemittel in die Speichereinrichtung dosierbar ist, wobei die Speichereinrichtung über einen Mitteldruckbypass (25) des Kühlkreislaufs an einer Mitteldruckseite (20) des Kühlkreislaufs in der Strömungsrichtung vor dem Hochdruckverdichter und nachfolgend dem Niederdruckverdichter angeschlossen ist,
wobei mittels eines Mitteldruckventils (26) des Kühlkreislaufs gasförmiges Kältemittel aus der Speichereinrichtung dosierbar ist,
**dadurch gekennzeichnet,**
**dass** das Kältemittel Kohlenstoffdioxid (CO₂) ist, wobei die Temperatur in einem Temperaturbereich von -20 °C bis +180 °C ausbildbar ist, wobei die Temperiervorrichtung eine Heizeinrichtung mit einer Heizung und einen Heiz-Wärmeübertrager in dem Prüfraum aufweist, wobei die Steuervorrichtung dazu eingerichtet ist, mittels des Mitteldruckventils gasförmiges Kältemittel in die Mitteldruckseite zu dosieren, wenn der Niederdruckverdichter abgeschaltet ist.

## Claims

1. A method for conditioning air in a temperature-insulated test space of a test chamber, which is sealable against an environment and serves for receiving test material, a temperature being produced within the test space by means of a cooling device (10) of a temperature control device of the test chamber, using a cooling circuit (11) with a cooling agent, using a heat exchanger (12) in the test space, using a low-pressure compressor (13) and using a high-pressure compressor (14) downstream of the low-pressure compressor, using a gas cooler (15), using a storage means (16) for the cooling agent and using an expansion valve (17), the temperature in the test space being controlled and/or regulated by means of a control device of the test chamber, the cooling agent being conducted past the low-pressure compressor to the low-pressure compressor via a bypass of the cooling circuit when the low-pressure compressor is switched off, a gaseous and/or liquid cooling agent being dosed in the storage means by means of a high-pressure valve (22) of the cooling circuit downstream of the gas cooler, the storage means being connected to a medium-pressure side (20) of the cooling circuit upstream of the high-pressure compressor and downstream of the low-pressure compressor via a medium-pressure bypass (25) of the cooling circuit,
**characterized in that**
the cooling agent is carbon dioxide (CO₂), the temperature being produced in a temperature range -20 °C to +180 °C, the temperature control device having a heating element having a heater and a thermal heat exchanger in the test space, the gaseous cooling agent being dosed in the medium-pressure side from the storage means by means of a medium-pressure valve (26) of the cooling circuit when the low-pressure compressor is switched off.

2. The method according to claim 1,
**characterized in that**
a temperature ranging from -40 °C to +180 °C, preferably from -55 °C to +180 °C, is produced within the test space by means of the temperature control device.

3. The method according to claim 1 or 2,
**characterized in that**
the low-pressure compressor (13) is switched off by the control device at a target temperature ranging from -10 °C to +180 °C, preferably from 0 °C to +180 °C.

4. The method according to any one of the preceding claims,
**characterized in that**
the cooling circuit (11) is operated in a thermodynamically subcritical, transcritical or supercritical operating state.

5. The method according to claim 4,
**characterized in that**
the high-pressure compressor (14) and the low-pressure compressor (13) are operated in at least the subcritical operating state.

6. The method according to claim 4 or 5,
**characterized in that**
in the supercritical state, the cooling agent expands at the high-pressure valve (22) and is guided to the storage means (16) as a gaseous cooling agent.

7. The method according to any one of the claims 4 to 6,
**characterized in that**
in the transcritical operating state, the high-pressure valve (22) is regulated in such a manner by means of the control device that the partially gaseous and liquid cooling agent is guided to the storage means (16).

8. The method according to any one of the claims 4 to 7,
**characterized in that**
a capacity of the high-pressure compressor (14) is adjusted as a function of a target temperature, the medium-pressure valve (26) being regulated in such a manner in the supercritical operating state by means of the control device that the gaseous cooling agent is guided to the medium-pressure side (20).

9. The method according to any one of the preceding claims,
**characterized in that**
a second medium-pressure bypass (28) having at least one second medium-pressure valve (29) is formed in the cooling circuit (11), the second medium-pressure bypass being connected to the cooling circuit downstream of the storage means (16) and upstream of the expansion valve (17) and downstream of the low-pressure compressor (13) and upstream of the high-pressure compressor (14), the liquid cooling agent being dosed in the medium-pressure side (20) from the storage means by means of the second medium-pressure valve.

10. The method according to any one of the preceding means,
**characterized in that**
a low-pressure bypass (30) having at least one second expansion valve (31) is disposed in the cooling circuit (11), the low-pressure bypass being connected to the cooling circuit downstream of the storage means (16) and upstream of the expansion valve (17) and downstream of the heat exchanger (12) and upstream of the low-pressure compressor (13), the liquid cooling agent being dosed in a low-pressure side (19) from the storage means by means of the second expansion valve.

11. The method according to any one of the preceding claims,
**characterized in that**
a regulating bypass (32) having at least one regulating valve (33) is disposed in the cooling circuit (11), the regulating bypass being connected to the cooling circuit downstream of the heat exchanger (12) and upstream of the low-pressure compressor (13) and downstream of the low-pressure compressor and upstream of the high-pressure compressor (14), the gaseous cooling agent being dosed in a low-pressure side (19) from the medium-pressure side (20) by means of the regulating valve, the low-pressure compressor being in operation.

12. The method according to claim 11,
**characterized in that**
the control device sets a suction pressure upstream of the low-pressure compressor (13) by means of the regulating bypass (32) in such a manner that the cooling agent is in a state below the triple-point upstream of the low-pressure compressor.

13. The method according to any one of the claims 1 to 10,
**characterized in that**
a regulating bypass (32) having at least one regulating valve (33) is formed in the cooling circuit (11), the regulating bypass being connected to the cooling circuit downstream of the heat exchanger (12) and upstream of the low-pressure compressor (13) and downstream of the low-pressure compressor and upstream of the high-pressure compressor (14), the gaseous cooling agent being dosed in the medium-pressure side (20) from a low-pressure side (19) by means of the regulating valve, the low-pressure compressor being switched off.

14. The method according to any one of the preceding claims,
**characterized in that**
the temperature control device comprises a dehumidifier which is made up of a dehumidifier bypass (34) having a dehumidifier valve (35) and an additional heat exchanger (37) in the test space, the dehumidifier bypass being connected to the storage means (16) and to the medium-pressure side (20) downstream of the low-pressure compressor (13) and upstream of the high-pressure compressor (14), the cooling agent being dosed in the medium-pressure side from the storage means by means of the dehumidifier valve when the low-pressure compressor is switched off or on.

15. The method according to claim 14,
**characterized in that**
the control device produces an evaporation temperature at the additional heat exchanger (37) which is lower than an evaporation temperature at the heat exchanger (12) by means of controlling the expansion valve (17) and/or the dehumidifier valve (35).

16. The method according to any one of the preceding claims,
**characterized in that**
pure carbon dioxide (CO₂) is used as the cooling agent.

17. A test chamber, in particular a climate chamber for conditioning air, the test chamber comprising a temperature-insulated test space, which is sealable against an environment and serves for receiving test material, and a temperature control device for controlling the temperature of the test space, a temperature being produced within the test space by means of the temperature control device, the temperature control device comprising a cooling device (10) having a cooling circuit (11) with a cooling agent, a heat exchanger (12) in the test space, a low-pressure compressor (13) and a high-pressure compressor (14) downstream of the low-pressure compressor, a gas cooler (15), a storage means (16) for the cooling agent and an expansion valve (17), the test chamber comprising a control device for controlling and/or regulating the temperature in the test space, the cooling agent being conducted past the low-pressure compressor to the high-pressure compressor via a bypass when the low-pressure compressor is switched off, the cooling circuit comprising a high-pressure valve (22) downstream of the gas cooler, the gaseous and/or liquid cooling agent being dosed in the storage means by means of the high-pressure valve, the storage means being connected to a medium-pressure side (20) of the cooling circuit upstream of the high-pressure compressor and downstream of the low-pressure compressor via a medium-pressure bypass (25) of the cooling circuit, the gaseous cooling agent being dosed from the storage means by means of a medium-pressure valve (26) of the cooling circuit,
**characterized in that**
the cooling agent is carbon dioxide (CO₂),the temperature being produced ranging from -20 °C to +180 °C, the temperature control device having a heating element having a heater and a thermal heat exchanger in the test space, the control device being configured to dose gaseous cooling agent in the medium-pressure side by means of the medium-pressure valve when the low-pressure compressor is switched off.

## Revendications

1. Procédé pour conditionner l'air dans un espace d'essai isolé thermiquement d'une chambre d'essai, qui peut être fermé de manière étanche par rapport à l'environnement et qui sert à recevoir un matériau d'essai, une température étant produite à l'intérieur de l'espace d'essai au moyen d'un refroidisseur (10) d'un dispositif de contrôle de la température et de la chambre d'essai, en utilisant un circuit de refroidissement (11) avec un fluide de refroidissement, en utilisant un échangeur de chaleur (12) dans l'espace d'essai, en utilisant un compresseur à basse pression (13) et en utilisant un compresseur à haute pression (14) en aval du compresseur à basse pression, en utilisant un refroidisseur de gaz (15), en utilisant un moyen de stockage (16) pour le fluide de refroidissement et en utilisant une soupape de détente (17), la température dans l'espace d'essai étant contrôlée et/ou régulée au moyen d'un dispositif de contrôle de la chambre d'essai, le fluide de refroidissement étant conduit devant le compresseur à basse pression vers le compresseur à haute pression par un bypass du circuit de refroidissement lorsque le compresseur à basse pression est arrêté, un fluide de refroidissement gazeux et/ou liquide étant dosé dans les moyens de stockage au moyen d'une soupape à haute pression (22) du circuit de refroidissement en aval du refroidisseur de gaz, les moyens de stockage étant reliés à un côté à moyenne pression (20) du circuit de refroidissement en amont du compresseur à haute pression et en aval du compresseur à basse pression par un bypass à moyenne pression (25) du circuit de refroidissement,
**caractérisé en ce que**
le fluide de refroidissement est du dioxyde de carbone (CO₂), la température étant produite dans une plage de température de -20 °C à +180 °C, le dispositif de contrôle de la température ayant un élément de chauffage ayant un chauffage et un échangeur de chaleur thermique dans l'espace d'essai, le fluide de refroidissement gazeux étant dosé dans le côté à moyenne pression à partir des moyens de stockage au moyen d'une soupape à moyenne pression (26) du circuit de refroidissement lorsque le compresseur à basse pression est arrêté.

2. Procédé selon la revendication 1,
**caractérisé en ce**
**qu'**une température comprise entre -40 °C et +180 °C, de préférence entre -55 °C et +180 °C, est produite dans l'espace d'essai au moyen du dispositif de contrôle de la température.

3. Procédé selon la revendication 1 ou la revendication 2,
**caractérisé en ce que**
le compresseur à basse pression (13) est désactivé par le dispositif de contrôle à une température cible comprise entre -10 °C et +180 °C, de préférence entre 0 °C et +180 °C.

4. Procédé selon l'une quelconque des revendications précédentes,
**caractérisé en ce que**
le circuit de refroidissement (11) est exploité dans un état de fonctionnement thermodynamiquement sous-critique, transcritique ou supercritique.

5. Procédé selon la revendication 4,
**caractérisé en ce que**
le compresseur à haute pression (14) et le compresseur à basse pression (13) sont exploités au moins dans l'état de fonctionnement sous-critique.

6. Procédé selon la revendication 4 ou la revendication 5,
**caractérisé en ce que**
dans l'état supercritique, le fluide de refroidissement se détend au niveau de la soupape à haute pression (22) et est guidé vers les moyens de stockage (16) sous forme de fluide de refroidissement gazeux.

7. Procédé selon l'une quelconque des revendications 4 à 6,
**caractérisé en ce que**
dans l'état de fonctionnement transcritique, la soupape à haute pression (22) est régulée au moyen du dispositif de contrôle de telle manière que le fluide de refroidissement partiellement gazeux et liquide est guidé vers les moyens de stockage (16).

8. Procédé selon l'une quelconque des revendications 4 à 7,
**caractérisé en ce**
**qu'**une capacité du compresseur à haute pression (14) est ajustée en fonction d'une température cible, la soupape à moyenne pression (26) étant régulée dans l'état de fonctionnement supercritique au moyen du dispositif de contrôle de telle manière que le fluide de refroidissement gazeux est guidé vers le côté à moyenne pression (20).

9. Procédé selon l'une quelconque des revendications précédentes,
**caractérisé en ce**
**qu'**un deuxième bypass à moyenne pression (28) ayant au moins une deuxième soupape à moyenne pression (29) est formé dans le circuit de refroidissement (11), le deuxième bypass à moyenne pression étant relié au circuit de refroidissement en aval des moyens de stockage (16) et en amont de la soupape de détente (17) et en aval du compresseur à basse pression (13) et en amont du compresseur à haute pression (14), le fluide de refroidissement liquide étant dosé dans le côté à moyenne pression (20) à partir des moyens de stockage au moyen du deuxième soupape à moyenne pression.

10. Procédé selon l'une quelconque des revendications précédentes,
**caractérisé en ce**
**qu'**un bypass à basse pression (30) ayant au moins une deuxième soupape de détente (31) est disposé dans le circuit de refroidissement (11), le bypass à basse pression étant relié au circuit de refroidissement en aval des moyens de stockage (16) et en amont de la soupape de détente (17) et en aval de l'échangeur de chaleur (12) et en amont du compresseur à basse pression (13), le fluide de refroidissement liquide étant dosé dans un côté à basse pression (19) à partir des moyens de stockage au moyen de la deuxième soupape de détente.

11. Procédé selon l'une quelconque des revendications précédentes,
**caractérisé en ce**
**qu'**un bypass de réglage (32) ayant au moins une soupape de réglage (33) est disposé dans le circuit de refroidissement (11), le bypass de réglage étant relié au circuit de refroidissement en aval de l'échangeur de chaleur (12) et en amont du compresseur à basse pression (13) et en aval du compresseur à basse pression et en amont du compresseur à haute pression (14), le fluide de refroidissement gazeux étant dosé dans un côté à basse pression (19) à partir du côté à moyenne pression (20) au moyen de la soupape de réglage, le compresseur à basse pression étant en fonctionnement.

12. Procédé selon la revendication 11,
**caractérisé en ce que**
le dispositif de contrôle règle une pression d'aspiration en amont du compresseur à basse pression (13) au moyen du bypass de réglage (32) de telle manière que le fluide de refroidissement se trouve dans un état inférieur au point triple en amont du compresseur à basse pression.

13. Procédé selon l'une quelconque des revendications 1 à 10,
**caractérisé en ce**
**qu'**un bypass de réglage (32) ayant au moins une soupape de réglage (33) est formé dans le circuit de refroidissement (11), le bypass de réglage étant relié au circuit de refroidissement en aval de l'échangeur de chaleur (12) et en amont du compresseur à basse pression (13) et en aval du compresseur à basse pression et en amont du compresseur à haute pression (14), le fluide de refroidissement gazeux étant dosé dans le côté à moyenne pression (20) à partir d'un côté à basse pression (19) au moyen de la soupape de réglage, le compresseur à basse pression étant désactivé.

14. Procédé selon l'une quelconque des revendications précédentes,
**caractérisé en ce que**
le dispositif de contrôle de la température comprend un déshumidificateur qui est constitué d'un bypass de déshumidificateur (34) ayant une soupape de déshumidificateur (35) et un échangeur de chaleur (37) supplémentaire dans l'espace d'essai, le bypass de déshumidificateur étant relié aux moyens de stockage (16) et au côté à moyenne pression (20) en aval du compresseur à basse pression (13) et en amont du compresseur à haute pression (14), le fluide de refroidissement étant dosé dans le côté à moyenne pression à partir des moyens de stockage au moyen de la soupape de déshumidificateur lorsque le compresseur à basse pression est désactivé ou mis en fonctionnement.

15. Procédé selon la revendication 14,
**caractérisé en ce que**
le dispositif de contrôle produit une température d'évaporation au niveau de l'échangeur de chaleur (37) supplémentaire qui est inférieure à une température d'évaporation au niveau de l'échangeur de chaleur (12) au moyen de la commande de la soupape de détente (17) et/ou de la soupape de déshumidificateur (35).

16. Procédé selon l'une quelconque des revendications précédentes,
**caractérisé en ce que**
du dioxyde de carbone pur (CO₂) est utilisé comme fluide de refroidissement.

17. Chambre d'essai, en particulier une chambre climatique pour conditionner l'air, la chambre d'essai comprenant un espace d'essai isolé thermiquement, qui peut être fermé de manière étanche par rapport à l'environnement et qui sert à recevoir le matériau d'essai, et un dispositif de contrôle de la température pour contrôler la température de l'espace d'essai, une température étant produite à l'intérieur de l'espace d'essai au moyen du dispositif de contrôle de la température, le dispositif de contrôle de la température comprenant un refroidisseur (10) ayant un circuit de refroidissement (11) avec un fluide de refroidissement, un échangeur de chaleur (12) dans l'espace d'essai, un compresseur à basse pression (13) et un compresseur à haute pression (14) en aval du compresseur à basse pression, un refroidisseur de gaz (15), un moyen de stockage (16) pour le fluide de refroidissement et une soupape de détente (17), la chambre d'essai comprenant un dispositif de contrôle pour contrôler et/ou réguler la température dans l'espace d'essai, le fluide de refroidissement étant conduit devant le compresseur à basse pression vers le compresseur à haute pression par un bypass du circuit de refroidissement lorsque le compresseur à basse pression est désactivé, le circuit de refroidissement comprenant une soupape à haute pression (22) en aval du refroidisseur de gaz, le fluide de refroidissement gazeux et/ou liquide étant dosé dans le moyen de stockage au moyen de la soupape à haute pression, les moyens de stockage étant reliés à un côté à moyenne pression (20) du circuit de refroidissement en amont du compresseur à haute pression et en aval du compresseur à basse pression par un bypass à moyenne pression (25) du circuit de refroidissement, le fluide de refroidissement gazeux étant dosé à partir des moyens de stockage au moyen d'une soupape à moyenne pression (26) du circuit de refroidissement,
**caractérisé en ce que**
le fluide de refroidissement est du dioxyde de carbone (CO₂), la température étant produite dans une plage comprise entre -20 °C à +180 °C, le dispositif de contrôle de la température comprenant un moyen de chauffage ayant un chauffage et un échangeur de chaleur thermique dans l'espace d'essai, le dispositif de contrôle étant configuré pour doser le fluide de refroidissement gazeux dans le côté à moyenne pression au moyen de la soupape à moyenne pression lorsque le compresseur à basse pression est désactivé.
